# FASCICULE DE BREVET EUROPEEN

(11) **EP 1 068 878 B1**
(45) Date de publication et mention de la délivrance du brevet: **15.06.2005**
(21) Numéro de dépôt: 00402025.1
(22) Date de dépôt: 13.07.2000
(51) Int. Cl.: A61N 1/365

(54) **Dispositif médical implantable actif, notamment stimulateur cardiaque, défibrillateur ou cardioverteur, asservi et protégé contre les effets des extrasystoles brady-et/ou tachy dépendantes**
Aktive implantierbare medizinische Vorrichtung, insbesondere Herzstimulator, Defibrillator oder Kardiovertierer, die gesteuert und vor den Auswirkungen von brady- und/oder tachyabhängigen Extrasystoles geschützt ist
Active implantable medical device, in particular pacemaker, defibrillator or cardioverter, which is controlled and protected against the effects of brady- and/or tachy-dependent extrasystoles

(30) Priorité: 15.07.1999 FR 9909165
(43) Date de publication de la demande: 17.01.2001
(73) Titulaire: ELA MEDICAL (Société anonyme), 92541 Montrouge (FR)
(72) Inventeur: Bonhour, Anne, 92410 Ville d'Avray (FR); Limousin, Marcel, 75014 Paris (FR); Bonnet, Jean-Luc, 92190 Montrouge (FR)
(74) Mandataire: Dupuis-Latour, Dominique

(56) Documents cités:
- EP-A- 0 672 433
- EP-A- 0 755 696
- US-A- 5 271 394
- US-A- 5 306 293
- US-A- 5 312 451

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement les conséquences que peuvent avoir les extrasystoles sur le fonctionnement de ces dispositifs.

Les extrasystoles peuvent être ventriculaires (ESV) ou auriculaires (ESA). Les ESV peuvent être de deux types : une ESV du premier type correspond à une détection ou stimulation ventriculaire non précédée d'un événement auriculaire (détection ou stimulation délivrée par le dispositif dans l'oreillette) dans un intervalle de temps considéré comme physiologique, par exemple compris entre 31 et 300 ms ; une ESV du second type correspond à une détection ventriculaire précédée d'un événement auriculaire dans un intervalle de temps compris entre 31 et 300 ms, dans le cas où le délai auriculo-ventriculaire du cycle examiné est inférieur de plus de 31 ms au délai auriculo-ventriculaire du cycle cardiaque précédent (le cycle cardiaque étant défini comme l'intervalle de temps entre deux événements de même nature dans la même cavité).

Une ESA correspond à une onde ou un événement P (le recueil d'une activité spontanée ayant son origine dans l'oreillette) suivant l'onde P du précédent événement auriculaire, avec un intervalle inférieur à une fraction de l'intervalle moyen de la fréquence auriculaire, calculée sur huit cycles cardiaques ne comprenant pas d'extrasystole.

En pratique, tous les patients présentent à l'état basal un certain nombre d'ESV ou ESA isolées, sans conséquence.

Mais quand ces extrasystoles deviennent trop fréquentes (typiquement, plus de dix ESV ou ESA par minute), ce phénomène peut altérer le remplissage des cavités, et donc la fonction hémodynamique du coeur, et/ou favoriser l'apparition de troubles du rythme.

La fréquence d'apparition de ces ESA ou ESV est déterminée par un paramètre appelé "taux d'extrasystolie", d'autant plus élevé que la fréquence d'apparition des ESA ou ESV est importante.

Pour certains patients, l'apparition d'une extrasystolie importante peut être liée à un niveau de la fréquence cardiaque soit trop bas (on parle alors d"'extrasystolie brady-dépendante"), soit trop élevé ("extrasystolie tachy-dépendante").

Le point de départ de l'invention réside dans la constatation du fait que, lorsque la fréquence de stimulation est déterminée par le dispositif en fonction de l'activité du patient, l'apparition d'extrasystoles brady-dépendantes ou tachy-dépendantes peut donner lieu à un phénomène indésirable d'oscillation et d'instabilité de l'algorithme d'asservissement de la fréquence de stimulation.

Ce phénomène apparaît notamment dans les dispositifs pourvus d'un asservissement de type "algorithme de fréquence de base de repos" tel que décrit par exemple dans le EP-A-0 672 433 (ELA Médical). Cet algorithme permet d'adapter la fréquence de base de stimulation à l'activité du patient, et en particulier de la baisser en cas de repos prolongé jusqu'à une fréquence minimale, appelée "fréquence de sommeil".

Lorsque le patient est sujet à des extrasystoles brady-dépendantes, on peut observer le phénomène de va-et-vient suivant :
- en période de repos prolongé, la fréquence du patient est abaissée jusqu'à la fréquence de sommeil ;
- cette fréquence basse déclenche des extrasystoles ;
- l'algorithme, qui possède une sécurité, diagnostique la présence d'une extrasystolie trop importante et remonte alors la fréquence, ce qui permet de quitter la zone favorisant l'extrasystolie ;
- mais, dans la mesure où le dispositif détecte toujours un repos prolongé, la fréquence est à nouveau abaissée, faisant par là-même revenir l'extrasystolie.

Un phénomène de va-et-vient semblable apparaît dans les phases d'effort, avec les dispositifs pourvus d'un asservissement à l'effort de la fréquence de stimulation.

Dans ces dispositifs, l'algorithme d'asservissement permet d'adapter la fréquence de stimulation à l'activité du patient, et en particulier de l'augmenter en cas d'effort. Si le patient présente une extrasystolie tachy-dépendante, cette accélération peut être un facteur favorisant l'apparition d'extrasystoles indésirables, ce qui va déclencher une sécurité et le retour à une fréquence plus basse, puis une nouvelle augmentation de la fréquence, et ainsi de suite.

Le but de l'invention est de proposer un dispositif permettant de détecter l'apparition de ces phénomènes indésirables et placer le dispositif dans un état empêchant qu'ils ne persistent.

Plus précisément, le dispositif de l'invention, qui est du type général décrit par exemple par le EP-A-0 672 433, comprend : des moyens d'analyse d'activité, aptes à évaluer le niveau d'activité du patient porteur du dispositif et à discriminer entre des phases de repos, d'activité normale et d'effort ; des moyens de stimulation d'au moins une cavité cardiaque, aptes à délivrer au coeur des impulsions électriques à une fréquence de base déterminée par le dispositif ; et des moyens aptes à ajuster ladite fréquence de base en fonction du niveau d'activité déterminé par les moyens d'analyse d'activité, cette fréquence de base pouvant être abaissée jusqu'à un niveau minimal donné pendant une phase de repos.

Pour détecter les extrasystolies brady-dépendantes et en pallier les conséquences, ce dispositif est caractérisé en ce qu'il comprend : des moyens de détection de la survenue d'extrasystoles et d'évaluation d'un taux d'extrasystolie correspondant ; et des moyens aptes à relever le niveau minimal de la fréquence de base lorsque le taux d'extrasystolie dépasse un seuil prédéterminé pendant ladite phase de repos. Ledit niveau minimal est de préférence ajusté à une valeur correspondant à un pas de fréquence juste supérieur à celui auquel les extrasystoles sont survenues. Avantageusement, le relèvement du niveau minimal de la fréquence de base est maintenu au moins pendant toute la durée de ladite phase de repos, et n'est de préférence pas rétabli à sa valeur antérieure avant détection d'une phase d'effort survenant après ladite phase de repos.

L'invention est également applicable, *mutatis mutandis,* à la détection des extrasystolies tachy-dépendantes et au traitement de leurs effets.

Pour ce faire, le dispositif de l'invention est alors caractérisé en ce qu'il comprend : des moyens de détection de la survenue d'extrasystoles et d'évaluation d'un taux d'extrasystolie correspondant ; et des moyens aptes à abaisser le niveau maximal de la fréquence asservie lorsque le taux d'extrasystolie dépasse un seuil prédéterminé pendant ladite phase d'effort. Ledit niveau maximal est de préférence ajusté à une valeur correspondant à un pas de fréquence juste inférieur à celui auquel les extrasystoles sont survenues.

Avantageusement, l'abaissement du niveau maximal de la fréquence asservie est maintenu au moins pendant toute la durée de ladite phase d'effort, et n'est de préférence pas rétabli à sa valeur antérieure avant détection d'une phase de repos survenant après ladite phase d'effort.

On va maintenant décrire un exemple de mise en oeuvre de l'invention, en référence aux dessins annexés.

La figure 1 illustre la manière dont l'invention permet de diagnostiquer et de traiter une extrasystolie brady-dépendante.

La figure 2 illustre la manière dont l'invention permet de diagnostiquer et de traiter une extrasystolie tachy-dépendante

De manière en elle-même connue, le dispositif surveille les événements survenant dans la cavité auriculaire ou ventriculaire du coeur et détecte l'apparition d'extrasystoles (ESA ou ESV) dans l'une ou l'autre de ces cavités. Le EP-A-0 755 696 (ELA Médical) décrit par exemple la manière de détecter de telles extrasystoles.

Le dispositif détermine également un taux d'extrasystolie en cas de survenue d'extrasystoles successives, ce taux étant par exemple défini comme le rapport, sur une durée donnée, entre le nombre d'extrasystoles détectées et le nombre de cycles cardiaques.

Le dispositif considérera qu'il y a présence effective d'une extrasystolie brady-dépendante (ou tachy-dépendante) lorsque le taux d'extrasystolie dépasse un seuil donné, typiquement plus de cinq extrasystoles sur 32 cycles cardiaques successifs. En variante, il est également possible de compter le nombre d'extrasystoles sur une période de temps donnée et de diagnostiquer l'extrasystolie lorsque le compteur atteint une valeur prédéterminée.

L'invention propose, essentiellement, de corréler le taux d'extrasystolie évalué par le dispositif à la fréquence de stimulation lorsque celle-ci varie en fonction de l'activité du patient.

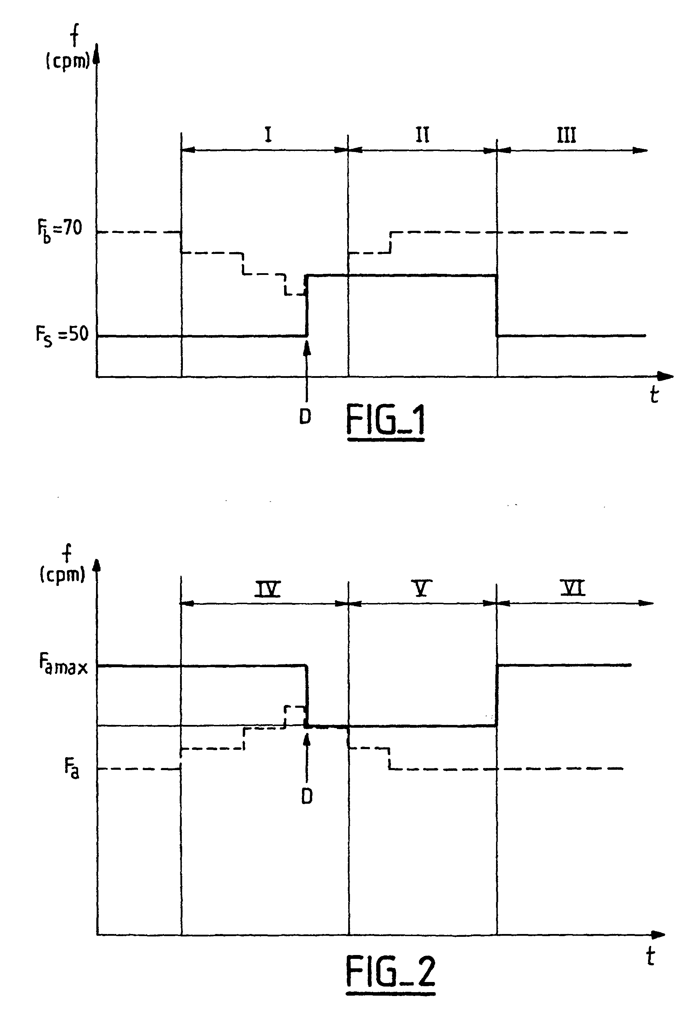

On va tout d'abord décrire, en référence à la figure 1, la manière dont sont traitées les extrasystolies brady-dépendantes.

Si l'on se réfère à la figure 1, la phase I correspond à une phase de repos prolongé du patient, typiquement une période de sommeil, diurne ou nocturne.

Une telle période de repos prolongé peut être par exemple diagnostiquée de façon automatique à partir du signal délivré par un capteur de suivi du rythme respiratoire du patient et/ou par un autre type de capteur, par exemple un capteur d'activité tel qu'un accéléromètre intégré au boîtier du dispositif ("capteur G").

On pourra se référer par exemple au EP-A-0 719 568 (Ela Médical) qui décrit un procédé de détermination d'un "critère d'activité de capteur" permettant d'opérer une distinction entre des phases de repos (nocturne ou diurne) du porteur de l'appareil et d'autres phases d'activité, notamment à partir d'un capteur de ventilation-minute, ou encore aux EP-A-0 750 920 (Ela Médical) et EP-A-0 770 407 (Ela Médical), qui décrivent des dispositifs médicaux utilisant les informations combinées d'un capteur physiologique et d'un capteur physique, notamment d'un capteur de ventilation-minute et d'un accéléromètre, pour déterminer un état d'activité ou de repos du patient.

Sur la figure 1, la phase I correspond à une période de repos ainsi déterminée par le dispositif. On peut noter qu'au fur et à mesure que ce repos se prolonge, la fréquence de base Fb est abaissée progressivement, depuis la valeur Fb = 70 cpm (par exemple) en tendant vers une valeur minimale programmée ou fréquence de sommeil Fs = 50 cpm (par exemple), qui constitue le minimum que peut atteindre la fréquence de base Fb. Cet abaissement progressif est opéré par exemple de la manière décrite dans le EP-A-0 672 433 précité.

Pendant ce temps, le dispositif continue à évaluer le taux d'extrasystolie. À l'instant D, correspondant à un diagnostic d'extrasystolie brady-dépendante avérée, le dispositif modifie la valeur de la fréquence minimale Fs à une valeur correspondant à un pas de fréquence juste supérieur à celui auquel les extrasystoles sont survenues, par exemple en remontant Fs à 60 cpm (au lieu de 50 cpm) si les extrasystoles sont survenues à 55 cpm et que le pas est de 5 cpm. De la sorte, la fréquence de base Fb ne pourra plus atteindre la fréquence génératrice d'extrasystoles.

Lorsque la phase I de repos prolongé s'achève et que le patient reprend une activité normale (phase II), la fréquence de base remonte normalement à sa valeur programmée, Fb = 70 cpm dans cet exemple.

Quant à la fréquence de sommeil Fs, maintenue à la nouvelle valeur (Fs = 60 cpm dans cet exemple), elle sera repositionnée à sa valeur antérieurement programmée (Fs = 50 cpm dans cet exemple) sur le prochain effort détecté par le dispositif (phase III).

En effet, le phénomène d'extrasystolie brady-dépendante est variable pour un même patient selon les circonstances (selon, par exemple que le repos correspond à une sieste ou à un sommeil nocturne), ou d'un jour ou d'une semaine à l'autre. L'ajustement de la fréquence de sommeil empêchant l'apparition d'extrasystoles brady-dépendantes ne sera donc pas nécessairement le même la prochaine fois qu'apparaîtra le phénomène.

L'invention est applicable de la même façon, *mutatis mutandis,* au traitement des extrasystoles tachy-dépendantes, illustré en référence à la figure 2.

De façon en elle-même connue, l'algorithme d'asservissement permet d'adapter la fréquence de stimulation (fréquence asservie Fa) à l'activité du patient, en particulier de l'augmenter en cas d'effort.

Cette fréquence est plafonnée à une valeur Faₘₐₓ paramétrée par le praticien au moment de l'implantation. En cas d'effort (phase IV), la fréquence asservie Fa augmente progressivement, sans pouvoir dépasser Faₘₐₓ.

Dans le cas d'un patient présentant une extrasystolie tachy-dépendante diagnostiquée à l'instant D, le dispositif va abaisser la fréquence maximale d'asservissement Faₘₐₓ au pas de fréquence juste inférieur à la valeur à laquelle sont survenues les extrasystoles.

Lorsque l'effort cesse et que le patient revient à une condition d'activité normale (phase V), la fréquence asservie Fa diminue corrélativement. Quant à la fréquence maximale d'asservissement Faₘₐₓ, une fois celle-ci abaissée, elle est maintenue à la nouvelle valeur pendant toute la durée de l'effort et n'est repositionnée à sa valeur initialement programmée que lorsqu'une phase de repos (phase VI) est détectée. En effet, comme dans le cas des extrasystolies brady-dépendantes, les extrasystolies tachy-dépendantes ne sont pas un phénomène systématique et répétitif, mais qui peut varier pour un même patient en fonction des conditions ou des périodes considérées.

## Revendications

1. Un dispositif médical actif implantable, notamment un stimulateur cardiaque, défibrillateur ou cardioverteur, comprenant :
- des moyens d'analyse d'activité, aptes à évaluer le niveau d'activité du patient porteur du dispositif et à discriminer entre des phases de repos (I), d'activité normale (II) et d'effort (III);
- des moyens de stimulation d'au moins une cavité cardiaque, aptes à délivrer au coeur des impulsions électriques à une fréquence de base déterminée par le dispositif ; et
- des moyens aptes à ajuster ladite fréquence de base en fonction du niveau d'activité déterminé par les moyens d'analyse d'activité, cette fréquence de base (Fb) pouvant être abaissée jusqu'à un niveau minimal donné (Fs) pendant une phase de repos (I) ;
dispositif **caractérisé en ce qu'**il comprend :
- des moyens de détection de la survenue d'extrasystoles et d'évaluation d'un taux d'extrasystolie correspondant ; et
- des moyens aptes à relever le niveau minimal de la fréquence de base lorsque le taux d'extrasystolie dépasse un seuil prédéterminé pendant ladite phase de repos.

2. Le dispositif de la revendication 1, dans lequel les moyens aptes à relever le niveau minimal de la fréquence de base sont des moyens aptes à maintenir le relèvement du niveau minimal de la fréquence de base au moins pendant toute la durée de ladite phase de repos (I).

3. Le dispositif de la revendication 2, dans lequel les moyens aptes à relever le niveau minimal de la fréquence de base sont des moyens aptes à fixer ledit niveau minimal à une valeur correspondant à un pas de fréquence juste supérieur à celui auquel les extrasystoles sont survenues.

4. Le dispositif de la revendication 2, dans lequel les moyens aptes à relever le niveau minimal de la fréquence de base sont des moyens aptes à ne pas rétablir le niveau minimal de la fréquence de base à sa valeur antérieure avant détection d'une phase d'effort (III) survenant après ladite phase de repos (I).

5. Un dispositif médical actif implantable, notamment un stimulateur cardiaque, défibrillateur ou cardioverteur, comprenant :
- des moyens d'analyse d'activité, aptes à évaluer le niveau d'activité du patient porteur du dispositif et à discriminer entre des phases de repos (VI), d'activité normale (V) et d'effort (IV) ;
- des moyens de stimulation d'au moins une cavité cardiaque, aptes à délivrer au coeur des impulsions électriques à une fréquence asservie déterminée par le dispositif ; et
- des moyens aptes à ajuster ladite fréquence asservie (Fa) en fonction du niveau d'activité déterminé par les moyens d'analyse d'activité, cette fréquence asservie pouvant être, accrue jusqu'à un niveau maximal donné (Faₘₐₓ) pendant une phase d'effort (IV) ;
dispositif **caractérisé en ce qu'**il comprend :
- des moyens de détection de la survenue d'extrasystoles et d'évaluation d'un taux d'extrasystolie correspondant ; et
- des moyens aptes à abaisser le niveau maximal de la fréquence asservie lorsque le taux d'extrasystolie dépasse un seuil prédéterminé pendant ladite phase d'effort.

6. Le dispositif de la revendication 5, dans lequel les moyens aptes à abaisser le niveau maximal de la fréquence asservie sont des moyens aptes à maintenir l'abaissement du niveau maximal de la fréquence asservie au moins pendant toute la durée de ladite phase d'effort (IV).

7. Le dispositif de la revendication 6, dans lequel les moyens aptes à abaisser le niveau maximal de la fréquence asservie sont des moyens aptes à fixer ledit niveau maximal à une valeur correspondant à un pas de fréquence juste inférieur à celui auquel les extrasystoles sont survenues.

8. Le dispositif de la revendication 6, dans lequel les moyens aptes à abaisser le niveau maximal de la fréquence asservie sont des moyens aptes à ne pas rétablir le niveau maximal de la fréquence asservie à sa valeur antérieure avant détection d'une phase de repos (VI) survenant après ladite phase d'effort (IV).

## Patentansprüche

1. Aktive implantierbare medizinische Vorrichtung, insbesondere ein Herzschrittmacher, Defibrillator oder Cardioverter, aufweisend:
- Mittel zur Analyse der Aktivität, angepasst an zur Analyse eines Aktivitätsniveaus eines Patienten, der die Vorrichtung trägt, und zur Unterscheidung zwischen den Phasen der Ruhe (I), der normalen Aktivität (II) und der Anstrengung (III);
- Mittel zur Stimulation wenigstens einer Herzkammer, angepasst zur Abgabe von elektrischen Impulsen an das Herz bei einer durch die Vorrichtung bestimmten Basisfrequenz; und
- Mittel, angepasst zur Einstellung der Basisfrequenz in Abhängigkeit vom Aktivitätsniveau, das durch die Mittel zur Analyse der Aktivität bestimmt wird, wobei die Basisfrequenz (Fb) abgesenkt werden kann bis auf ein minimales gegebenes Niveau (Fs) während einer Ruhephase (I);
wobei die Vorrichtung **dadurch gekennzeichnet ist, dass** sie aufweist:
- Mittel zur Detektion des Auftretens von Extrasystolen und zur Berechnung einer entsprechenden extrasystolischen Rate; und
- Mittel, angepasst zur Feststellung eines minimalen Basisfrequenzniveaus, wenn die extrasystolische Rate eine vorbestimmte Schwelle während der Ruhephase überschreitet.

2. Vorrichtung nach Anspruch 1, bei der die Mittel, die zur Feststellung des minimalen Basisfrequeazniveaus angepasst sind, Mittel sind, die daran angepasst sind, die Feststellung des Basisfrequenzniveaus wenigstens während der gesamten Dauer der Ruhephase (I) beizubehalten.

3. Vorrichtung nach Anspruch 2, bei der die Mittel, die zur Feststellung des minimalen Basisfrequenzniveaus angepasst sind, Mittel sind, die angepasst sind, das minimale Niveau auf einen entsprechenden Wert festzulegen, der einem Frequcnzschritt entspricht, gerade oberhalb demjenigen, auf dem die Extrasystolen aufgetreten sind.

4. Vorrichtung nach Anspruch 2, bei der die Mittel, die zur Feststellung des minimalen Basisfrequenzniveaus angepasst sind, Mittel sind, die angepasst sind, kein minimales Basisfrequenzniveau auf seinen vorangehenden Wert wiederherzustellen, vor der Detektion einer Anstrengungsphase (III), die nach der Ruhephase (I) auftritt.

5. Aktive implantierbare medizinische Vorrichtung, insbesondere ein Herzschrittmacher, Defibrillator oder Cardioverter, aufweisend:
- Mittel zur Analyse der Aktivität, angepasst zur Berechnung des Aktivitätsniveaus des Patienten, der die Vorrichtung trägt, und zur Unterscheidung zwischen den Phasen der Ruhe (VI), der normalen Aktivität (V) und der Anstrengung (IV);
- Mittel zur Stimulation wenigstens einer Herzkammer, angepasst zur Abgabe von elektrischen Impulsen an das Herz, bei einer Regelfrequenz, welche durch die Vorrichtung festgelegt wird; und
- Mittel, angepasst zur Einstellung der Regelfrequenz (Fa) abhängig vom Aktivitätsniveau, welches durch die Mittel zur Analyse der Aktivität bestimmt wird, wobei die Regelfrequenz bis auf ein maximales gegebenes Niveau (Faₘₐₓ) während einer Anstrengungsphase (IV) angehoben werden kann;
wobei die Vorrichtung **dadurch gekennzeichnet ist, dass** sie aufweist:
- Mittel zur Detektion des Auftretens von Extrasystolen und zur Auswertung einer entsprechenden extrasystolischen Rate; und
- Mittel, geeignet zur Absenkung des maximalen Regelfrequenzniveaus, wenn die extrasystolische Rate eine vorbestimmte Schwelle während der Anstrengungsphase überschreitet.

6. Vorrichtung nach Anspruch 5, bei der die Mittel, welche geeignet sind, das maximale Regelfrequenzniveau abzusenken, Mittel sind zur Beibehaltung der Absenkung des maximalen Regelfrequenzniveaus, wenigstens während der gesamten Dauer der Anstrengungsphase (IV).

7. Vorrichtung nach Anspruch 6, bei der die Mittel, geeignet zur Absenkung des maximalen Regelfrequenzniveaus, Mittel sind, die geeignet sind zur Festlegung des maximalen Niveaus auf einen Wert, der einem Frequenzschritt entspricht, der gerade unterhalb desjenigen liegt, bei dem die Extrasystolen aufgetreten sind.

8. Vorrichtung nach Anspruch 6, bei der die Mittel, die dazu geeignet sind, das maximale Regelfrequenzniveau abzusenken, Mittel sind, die dazu geeignet sind, das maximale Regelfrequenzniveau nicht vor der Detektion einer Ruhephase (VI), die nach der Anstrengungsphase (IV) auftritt, auf seinen vorhergehenden Wert wiederherstellen.

## Claims

1. An active implantable medical device, in particular a cardiac pacemaker, defibrillator or cardioverter, including:
- activity analysis means, adapted to evaluate the activity level of the patient bearing the device and to discriminate between phases of rest (I), normal activity (II) and effort (III);
- means for stimulating at least one cardiac cavity, adapted to deliver to the heart electrical pulses at a base frequency determined by the device; and
- means adapted to adjust said base frequency according to the activity level determined by the activity analysis means, said base frequency (Fb) being possibly decreased down to a given minimal level (Fs) during a rest phase (I);
said device being **characterised by** comprising :
- means for detecting the occurrence of extrasystoles and for evaluating a corresponding extrasystole rate; and
- means adapted to raise the minimal level of the base frequency when the extrasystole rate is above a predetermined threshold during said rest phase.

2. The device of claim 1, wherein the means adapted to raise the minimal level of the base frequency are means adapted to maintain the raise of the minimal level of the base frequency at least for the duration of said rest phase (I).

3. The device of claim 2, wherein the means adapted to raise the minimal level of the base frequency are means adapted to set said minimum level to a value just corresponding to one frequency step above the one at which the extrasystoles occurred.

4. The device of claim 2, wherein the means adapted to raise the minimal level of the base frequency are means adapted to restore the minimal level of the base frequency to its prior value not before the detection of an effort phase (III) occurring after said rest phase (I).

5. An active implantable medical device, in particular a cardiac pacemaker, defibrillator or cardioverter, including:
- activity analysis means, adapted to evaluate the activity level of the patient bearing the device and to discriminate between phases of rest (VI), normal activity (V) and effort (IV);
- means for stimulating at least one cardiac cavity, adapted to deliver to the heart electrical pulses at an enslaved frequency determined by the device; and
- means adapted to adjust said enslaved frequency (Fa) according to the activity level determined by the activity analysis means, said base frequency being possibly increased up to a given maximal level (Faₘₐₓ) during an effort phase (IV);
said device being **characterised by** comprising :
- means for detecting the occurrence of extrasystoles and for evaluating a corresponding extrasystole rate; and
- means adapted to decrease the maximal level of the enslaved frequency when the extrasystole rate is above a predetermined threshold during said effort phase.

6. The device of claim 5, wherein the means adapted to decrease the maximal level of the enslaved frequency are means adapted to maintain the increase of the maximal level of the enslaved frequency at least for the duration of said effort phase (IV).

7. The device of claim 6, wherein the means adapted to decrease the maximal level of the enslaved frequency are means adapted to set said maximum level to a value just corresponding to one frequency step below the one at which the extrasystoles occurred.

8. The device of claim 6, wherein the means adapted to decrease the maximal level of the enslaved frequency are means adapted to restore the maximal level of the enslaved frequency to its prior value not before the detection of a rest phase (VI) occurring after said effort phase (IV).
